# EUROPÄISCHE PATENTSCHRIFT

(11) **EP 1 663 944 B1**
(45) Veröffentlichungstag und Bekanntmachung des Hinweises auf die Patenterteilung: **07.03.2007**
(21) Anmeldenummer: 04764835.7
(22) Anmeldetag: 04.09.2004
(51) Int. Cl.: C07C 209/48, C07C 211/27

(54) **VERFAHREN ZUR HERSTELLUNG VON XYLYLENDIAMIN (XDA)**
METHOD FOR PRODUCING XYLYLENEDIAMINE (XDA)
PROCEDE DE PRODUCTION DE XYLYLENE DIAMINE (XDA)

(30) Priorität: 10.09.2003 DE 10341633
(43) Veröffentlichungstag der Anmeldung: 07.06.2006
(73) Patentinhaber: BASF Aktiengesellschaft, 67056 Ludwigshafen (DE)
(72) Erfinder: HUGO, Randolf, 67246 Dirmstein (DE); JOURDAN, Sabine, 68163 Mannheim (DE); WENZ, Kirsten, 68161 Mannheim (DE); PREISS, Thomas, 67256 Weisenheim (DE); WECK, Alexander, D-67251 Freinsheim (DE)
(86) Internationale Anmeldenummer: PCT/EP2004/009884
(87) Internationale Veröffentlichungsnummer: WO 2005/026103

(56) Entgegenhaltungen:
- EP-A- 1 193 244
- EP-A- 1 279 661

## Beschreibung

Die vorliegende Erfindung betrifft ein Verfahren zur Herstellung von Xylylendiamin umfassend die Schritte Ammonoxidation von Xylol zu Phthalodinitril und Hydrierung des Phthalodinitrils.

Xylylendiamin (Bis(aminomethyl)benzol) ist ein nützlicher Ausgangsstoff, z.B. für die Synthese von Polyamiden, Epoxyhärtern oder als Zwischenstufe zur Herstellung von Isocyanaten.

Die Bezeichnung "Xylylendiamin" (XDA) umfasst die drei Isomere ortho-Xylylendiamin, meta-Xylylendiamin (MXDA) und para-Xylylendiamin.

Der Begriff "Phthalodinitril" (PDN) umfasst die drei Isomere 1,2-Dicyanbenzol = o-Phthalodinitril, 1,3-Dicyanbenzol = Isophthalodinitril = IPDN und 1,4-Dicyanbenzol = Terephthalodinitril.

Die zweistufige Synthese von Xylylendiamin durch Ammonoxidation von Xylol und anschließender Hydrierung des erhaltenen Phthalodinitrils ist bekannt.

EP-A2-1 113 001 (Mitsubishi Gas Chem. Comp.) beschreibt ein Verfahren zur Herstellung von Nitrilverbindungen durch Ammonoxidation entsprechender carbocyclischer oder heterocyclischer Verbindungen, wobei überschüssiger Ammoniak aus dem Reaktionsprodukt recycliert wird. Beschrieben wird auch das direkte in Kontakt bringen des dampfförmigen Produkts der Ammonoxi dationsstufe mit einem flüssigen organischen Lösungsmittel, bei dem es sich insbesondere um aliphatische oder aromatische Kohlenwasserstoffe handelt. (Absätze [0045] und [0046]).

EP-A2-1 193 247 und EP-A1-1 279 661 (beide Mitsubishi Gas Chem. Comp.) betreffen ein Verfahren zur Reinigung von Isophthalodinitril (IPDN) bzw. ein Verfahren zur Herstellung von reinem XDA, in dem das Phthalodinitril durch Ammonoxidation von Xylol synthetisiert wird, wobei das dampfförmige Produkt der Ammonoxidationsstufe direkt mit einem flüssigen organischen Lösungsmittel in Kontakt gebracht wird (Quench).

Das organische Lösungsmittel ist ausgewählt aus Alkylbenzole, heterocyclische Verbindungen, aromatische Nitrile und heterocyclische Nitrile und hat einen Siedepunkt, der unter dem von Phthalodinitril liegt (EP-A2-1 193 247: Spalte 4, Absatz [0018] und [0019]; EP-A1-1 279 661: Spalten 4-5, Absatz [0023] und [0024]).

EP-A2-1 193 244 (Mitsubishi Gas Chem. Comp.) beschreibt ein Verfahren zur Herstellung von XDA durch Hydrierung von Phthalodinitril, welches in einer vorherigen Stufe durch Ammonoxidation von Xylol synthetisiert wird, wobei das dampfförmige Produkt der Ammonoxidationsstufe direkt mit einem flüssigen organischen Lösungsmittel in Kontakt gebracht wird (Quench) und die erhaltene Quenchlösung oder -suspension der Hydrierung zugeführt wird.
Bevorzugte organische Lösungsmittel sind C₆-C₁₂ aromatische Kohlenwasserstoffe, wie Xylol und Pseudocumol (Spalte 6, Absatz [0027] und [0028]).

DE-A-21 64 169 beschreibt auf Seite 6, letzter Absatz, die Hydrierung von IPDN zu meta-XDA in Gegenwart eines Ni- und/oder Co-Katalysators in Ammoniak als Lösungsmittel.

Fünf parallele BASF-Patentanmeldungen mit jeweils gleichem Anmeldetag betreffen jeweils Verfahren zur Herstellung von XDA.

Der vorliegenden Erfindung lag die Aufgabe zugrunde, ein verbessertes wirtschaftliches Verfahren zur Herstellung von hoch reinem Xylylendiamin, insbesondere meta-Xylylendiamin, mit hoher Ausbeute und Raum-Zeit-Ausbeite (RZA) aufzufinden, welches bei mit Verfahren des Stands der Technik (z.B. EP-A2-1 193 244, EP-A1-1 279 661) vergleichbaren Durchsätzen aufgrund verringerter Stoffströme, insbesondere Lösungsmittelströme, inkl. Rückführströme, verkleinerte und/oder weniger Apparate und Maschinen ermöglicht.

Demgemäß wurde ein Verfahren zur Herstellung von Xylylendiamin umfassend die Schritte Ammonoxidation von Xylol zu Phthalodinitril und Hydrierung des Phthalodinitrils gefunden,
welches dadurch gekennzeichnet ist, dass das dampfförmige Produkt der Ammonoxidationsstufe direkt mit einem flüssigen organischen Lösungsmittel welches einen niedrigeren Siedepunkt als das Phthalodinitril hat, oder mit geschmolzenem Phthalodinitril in Kontakt gebracht wird (Quench),
aus der erhaltenen Quenchlösung oder -suspension bzw. Phthalodinitrilschmeize Komponenten mit einem Siedepunkt niedriger als Phthalodinitril (Leichtsieder) teilweise oder vollständig abgetrennt werden und
vor der Hydrierung des Phthalodinitrils keine Produkte mit einem Siedepunkt höher als Phthalodinitril (Hochsieder) abgetrennt werden und die Hydrierung in Abwesenheit eines organischen Lösungsmittels durch geführt wird.

Bevorzugt findet das erfindungsgemäße Verfahren Anwendung zur Herstellung von meta-Xylylendiamin (MXDA) durch Hydrierung von Isophthalodinitril (IPDN), welches in einer vorherigen Stufe durch Ammonoxidation von meta-Xylol synthetisiert wird.

Das erfindungsgemäße Verfahren lässt sich wie folgt ausführen:

### Ammonoxidationsstufe:

Die Ammonoxidation von Xylol (o-, m- oder p-Xylol) zum entsprechenden Phthalodinitril (ortho-Xylol → o-Phthalodinitril; meta-Xylol → Isophthalodinitril; para-Xylol → Terephthalodinitril) wird im allgemeinen nach dem Fachmann bekannten Verfahren durchgeführt.

Die Ammonoxidation des Methylaromaten wird bevorzugt durchgeführt an einem Multioxidkatalysator mit Ammoniak und einem sauerstoffhaltigen Gas (Sauerstoff oder Luft oder beides) in einem Wirbelschichtreaktor oder einem Rohr(bündel)reaktor.

Die Reaktionstemperatur liegt dabei im allgemeinen bei 300 bis 500°C, bevorzugt bei 330 bis 480°C.

Der Katalysator enthält bevorzugt V, Sb und/oder Cr und setzt sich besonders bevorzugt zusammen aus [V, Sb und Alkalimetallen] oder [V, Cr, Mo und B] jeweils als Vollkatalysator oder auf einem inerten Träger.
Als inerter Träger sind bevorzugt SiO₂, Al₂O₃ oder ein Gemisch der beiden oder Steatit.

Solch eine Verfahrensweise ist z.B. in den BASF-Patentanmeldungen EP-A-767 165 und EP-A-699 476 beschrieben, auf die hiermit ausdrücklich Bezug genommen wird.

Auch die BASF-Patentanmeldungen EP-A-222 249, DE-A-35 40 517 und DE-A-37 00 710 offenbaren geeignete Ammonoxidationskatalysatoren.

Die Ammonoxidation kann auch gemäß den in den eingangs zitierten Anmeldungen EP-A2-1 113 001, EP-A2-1 193 247, EP-A1-1 279 661 und EP-A2-1 193 244 beschriebenen Verfahren durchgeführt werden.

### Quench:

Der bei der Ammonoxidation produzierte Dampf, enthaltend das Wertprodukt Phthalodinitril, wird direkt mit einem flüssigen organischen Lösungsmittel oder mit flüssigem, also geschmolzenem, Phthalodinitril (bevorzugt dasjenige Isomer, das dem synthetisierten PDN entspricht) in Kontakt gebracht (Quench mit einem flüssigen organischen Lösungsmittel oder mit geschmolzenem Phthalodinitril als Quenchflüssigkeit, Quenchmittel).

Das für den Quench verwendete organische Lösungsmittel kann auch bereits gelöstes oder suspendiertes Phthalodinitril (bevorzugt dasjenige Isomer, das dem synthetisierten PDN entspricht) enthalten.

Bevorzugte organische Lösungsmittel für den Quench sind ausgewählt aus der Gruppe aromatische Kohlenwasserstoffe (insbesondere Alkylaromaten, ganz besonders Alkylbenzole), heterocyclische Verbindungen, aromatische Nitrile und heterocyclische Nitrile und Mischungen hiervon.

Beispiele für solche verwendbaren Lösungsmittel sind o-Xylol, m-Xylol, p-Xylol, Pseudocumol, Mesitylen, Ethylbenzol, Methylpyridin, Benzonitril, m-Tolunitril, o-Tolunitril, p-Tolunitril, N-Methyl-2-pyrrolidon (NMP), THF, Methanol und 1,4-Dioxan.

Als organisches Lösungsmittel besonders bevorzugt sind Tolunitril, Benzonitril und NMP und Mischungen hiervon.

Das organische Lösungsmittel für den Quench hat einen niedrigeren Siedepunkt als das synthetisierte PDN (bei gleichem Druck).

Durch die plötzliche Temperaturabsenkung beim in Kontakt bringen des dampfförmigen Phthalodinitrils mit dem flüssigen Lösungsmittel oder mit dem geschmolzenem Phthalodinitril (Quench) wird die Bildung von unerwünschten Neben- und Zersetzungsprodukten, die zur Qualitätsminderung des Phthalodinitrils und schließlich des XDAs führen, verringert.

Das dampfförmige Phthalodinitril wird durch den Quench direkt in das flüssige organische Lösungsmittel oder das geschmolzene Phthalodinitril aufgenommen, wobei im Falle des flüssigen organischen Lösungsmittels eine Lösung und/oder eine Suspension und im Falle des geschmolzenen Phthalodinitrils eine Phthalodinitrilschmelze enthaltend das synthetisierte PDN entsteht.

Das organische Lösungsmittel für den Quench bzw. das geschmolzene Phthalodinitril für den Quench kann als Frischzulauf mit einer Reinheit > 99 Gew.-%, insbesondere > 99,5 Gew.-%, eingesetzt werden.

Bevorzugt kann aus dem Verfahren zurückgewonnenes organisches Lösungsmittel oder verfahrensgemäß hergestelltes Phthalodinitril als Quenchflüssigkeit eingesetzt werden. Hier kann die Reinheit der Quenchflüssigkeit auch ≤ 99 Gew.-%, z.B. 90 - 98 Gew.-%, betragen, insbesondere dann, wenn es sich nicht um verfahrensfremde Substanzen (also u.a. um Wasser, Ammoniak, Benzonitril, Tolunitril, Xylol, o-, m- oder p-Methyl-benzylamin, Benzylamin, Xylylendiamin) als Verunreinigungen handelt.

Die Menge des verwendeten organischen Lösungsmittels ist im allgemeinen so bemessen, dass Lösungen/Suspensionen mit einem Phthalodinitril-Gehalt von 15 bis 75 Gew.-%, bevorzugt 25 bis 60 Gew.-%, erhalten werden.

Im Fall von geschmolzenem Phthalodinitril als Quenchmittel richtet sich die Menge des verwendeten geschmolzenen Phthalodinitrils im wesentlichen nach der im Quench abzuführenden Wärme.

Die Einleitung des dampfförmigen Austrags der Ammonoxidation, enthaltend das Phthalodinitril (PDN), in das flüssige organische Lösungsmittel bzw. in das geschmolzene Phthalodinitril erfolgt in einem Quenchapparat, z.B. bevorzugt in einem Fallfilmkondensator (Dünnschicht-, Rieselfilm- oder Fallstromkondensator), in einem Düsenapparat oder in einer Kolonne. Dabei kann das dampfförmige Phthalodinitril im Gleich- oder im Gegenstrom mit dem flüssigen Lösungsmittel bzw. dem geschmolzenen Phthalodinitril geführt werden. Bei Gleichstromführung wird das dampfförmige Phthalodinitril von oben in den Quenchapparat eingeleitet. Vorteilhaft ist die tangentiale Zufuhr des flüssigen Lösungsmittels bzw. geschmolzenen Phthalodinitrils am Kopf des Fallfilmkondensators oder die Zufuhr des flüssigen Lösungsmittels bzw. geschmolzenen Phthalodinitrils durch eine oder mehrere Düsen um eine vollständige Benetzung der Innenwand des Quenchapparates zu erreichen.

Der Quenchapparat kann zur Vergrößerung der zur Kondensation verfügbaren Oberfläche mit Einbauten wie Böden, geordneten Packungen oder ungeordneten Schüttungen ausgerüstet sein.

Das organische Lösungsmittel bzw. geschmolzene Phthalodinitril für den Quench kann im einmaligen Durchlauf oder als Kreislaufmedium eingesetzt werden. Vorteilhafterweise wird ein Teil der Quenchlösung oder -Suspension bzw. Phthalodinitrilschmelze im Kreis gefahren (Kreislauf).

Mittels eines im Kreislauf eingebauten Wärmeüberträgers wird die Quenchlösung oder -suspension bzw. Phthalodinitrilschmelze gekühlt.
Dabei werden die Temperatur des Kreislaufmediums und der Kreislaufmengenstrom so eingestellt und aufeinander abgestimmt, dass die gewünschte Temperatur im Quenchaustritt erreicht wird. Die Temperatur des Kreislaufmediums wird umso niedriger gewählt, je kleiner der Mengenstrom des Kreislaufmediums ist und umgekehrt, wobei Löslichkeiten und Schmelzpunkte sowie die hydraulischen Belastungsgrenzen des Quenchapparates zu berücksichtigen sind.

Der Mengenstrom des frisch zulaufenden organischen Lösungsmittels ist von der Quenchtemperatur abhängig. Er wird so eingestellt, dass die gewünschte Konzentration der PDN-Lösung oder -suspension erhalten wird. Da die Löslichkeit von PDN im organischen Lösungsmittel mit zunehmender Temperatur ansteigt, kann mit zunehmender Quenchaustrittstemperatur eine höhere PDN-Konzentration im Lösungsmittel gefahren werden.

Das Kreislaufmedium bzw. das geschmolzene Phthalodinitril wird gemeinsam mit dem frischen Lösungsmittel oder davon getrennt an geeigneter Stelle des Quenchapparats zugefahren.

Im allgemeinen wird durch Temperierung des eingesetzten organischen Lösungsmittels und/oder des Kreislaufmediums die Temperatur des flüssigen Quenchaustrags eingestellt, und zwar auf 40 bis 180°C, bevorzugt 50 bis 120°C, insbesondere 80 bis 120°C.

Im Fall von geschmolzenem Phthalodinitril als Quenchmittel wird durch Temperierung des eingesetzten geschmolzenen Phthalodinitrils und/oder des Kreislaufmediums die Temperatur des flüssigen Quenchaustrags eingestellt, und zwar auf 165 bis 220°C, bevorzugt 180 bis 220°C, insbesondere 190 bis 210°C.
Der Absolutdruck beim Quenchen beträgt im allgemeinen 0,5 bis 1,5 bar. Bevorzugt wird bei leichtem Überdruck gefahren.

Xylol, Wasser, NH₃, CO₂, N₂ etc., die im dampfförmigen Austrag der Ammonoxidation in der Regel enthalten sind, werden unter Quenchbedingungen im Quenchmittel (organisches Lösungsmittel bzw. geschmolzenes Phthalodinitril) nur teilweise oder praktisch nicht gelöst und werden aus dem Quench-Apparat überwiegend gasförmig abgetrennt.

Teilweise oder vollständige Abtrennung von Komponenten mit einem Siedepunkt niedriger als Phthalodinitril (Leichtsieder) (bei gleichem Druck) aus der erhaltenen Quenchlösung oder -suspension bzw. Phthalodinitrilschmelze:

Je niedriger die Temperatur im Quenchschritt ist, desto höher ist der Anteil von Wasser und tiefer als PDN siedenden Nebenkomponenten (bei gleichem Druck) (z.B. Benzonitril, Tolunitril) im flüssigen Quenchaustrag.

Im erfindungsgemäßen Verfahren werden vor der Hydrierung des Phthalodinitrils aus der erhaltenen Quenchlösung oder -suspension bzw. Phthalodinitrilschmelze Wasser und Komponenten mit einem Siedepunkt niedriger als Phthalodinitril (bei gleichem Druck) (Leichtsieder; z.B. nicht umgesetztes Xylol, Benzonitril, Tolunitril, jeweils als Heteroazeotrop mit Wasser, Wasser, Benzonitril, Tolunitril; Aufzählung mit zunehmenden Siedepunkt (bei gleichem Druck); wie ggf. auch Benzylamin, o-, m-, p-Methyl-benzylamin, Xylylendiamine, wobei diese Amine aus zurückgeführten Lösungsmittel von der Hydrierstufe stammen) teilweise oder vollständig abgetrennt. Diese Abtrennung erfolgt bevorzugt destillativ.

Bevorzugt wird auch das im Quench verwendete organische Lösungsmittel in diesem Schritt als Leichtsieder teilweise oder vollständig abgetrennt.

Diese Abtrennung des Lösungsmittels und/oder der Leichtsieder kann in einer oder mehreren hintereinander geschalteten Verdampferstufen oder in einer Destillationskolonne über Kopf erfolgen, während Phthalodinitril zusammen mit Produkten mit einem Siedepunkt höher als Phthalodinitril (Hochsieder) (bei gleichem Druck) über Sumpf abgetrennt werden.

Bevorzugt wird eine Destillationskolonne verwendet, welche vorzugsweise mit den üblichen Einbauten zur Erhöhung der Trennleistung, wie Böden, geordnete oder ungeordnete Packungen, etc., ausgerüstet ist.

Die Auslegung der Kolonne (insbesondere Zahl der Trennstufen, Zulaufstelle, Rücklaufverhältnis, etc.) kann, abgestimmt auf die jeweilige Zusammensetzung der Lösung oder Suspension, durch den Fachmann nach ihm geläufigen Methoden vorgenommen werden.
Bevorzugt wird unter vermindertem Druck gefahren, um die Sumpftemperatur zu begrenzen.

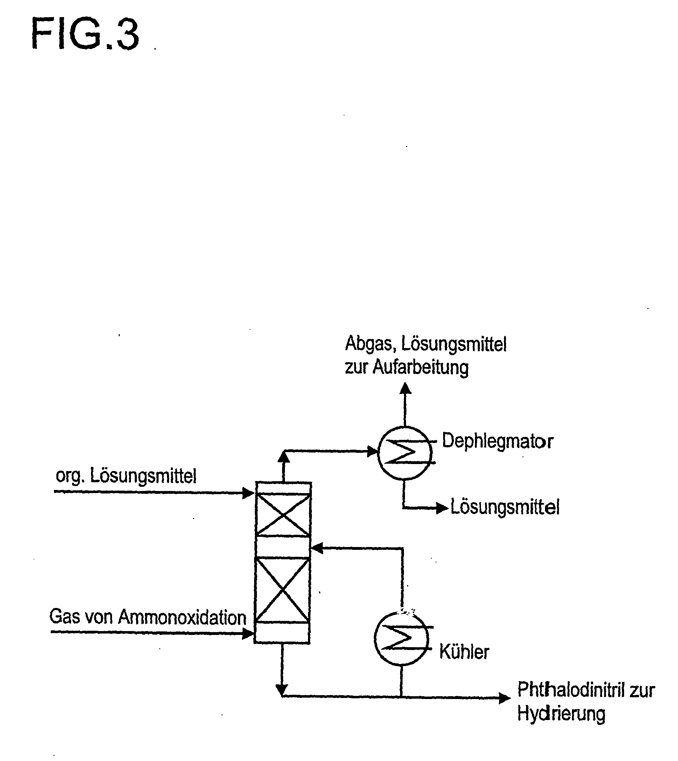

In einer besonderen Ausgestaltung des erfindungsgemäßen Verfahrens wird der Quench des dampfförmigen Produkts der Ammonoxidationsstufe mit einem flüssigen organischen Lösungsmittel oder mit geschmolzenem Phthalodinitril so in einer Kolonne durchgeführt, dass Reaktionsgase und Leichtsieder, inkl. dem ggf. verwendeten organischen Lösungsmittel als Quenchmittel, teilweise oder vollständig über Kopf und Phthalodinitril zusammen mit Produkten mit einem Siedepunkt höher als Phthalodinitril (Hochsieder) über Sumpf abgetrennt werden. (Vgl. Abbildung 3).

Mit dieser besonderen Verfahrensweise werden Quench und Leichtsiederabtrennung in einer Stufe (einem Schritt) zusammengefasst und in einem speziellen Quenchapparat, einer Quenchkolonne, durchgeführt. Besonders vorteilhaft ist wieder die bereits oben beschriebene Kreislauffahrweise eines Teils des Quenchkolonnenaustrags, wobei die Rückführung als Quenchmittel bevorzugt etwa in die Kolonnenmitte erfolgt.

Anschließend können ggf. noch vorhandene Leichtsieder in einem nachgeschalteten weiteren Schritt bei vermindertem Druck durch Verdampfung oder Rektifikation vollständig aus dem erhaltenen Phthalodinitril abgetrennt werden. Bevorzugt ist, dass in dieser Verfahrensweise keine weitere Leichtsiederabtrennung mehr durchgeführt wird, sondern die erhaltene PDN-Schmelze vom kombinierten Quench-/Leichtsiederabtrennungs-Schritt zur Hydrierung gefahren wird.

Der gasförmige Austrag aus der Ammonoxidation wird bevorzugt am Kolonnensumpf der Quenchkolonne aufgegeben und das frische Quenchmittel (organisches Lösungsmittel) am Kopf (Gegenstrom), während der Quenchkolonnenaustrag aus einem Gemisch von Lösungsmittel und PDN besteht oder (je nach gewählter Temperatur) praktisch lösungsmittelfrei ist (vgl. Abb. 3). Die Zusammensetzung des Quenchkolonnenaustrags wird durch die Betriebsbedingungen der Quenchkolonne (insbesondere Temperatur) und dem Mengenstrom des am Kopf der Quenchkolonne zugeführten Lösungsmittels festgelegt.

Im Falle, dass PDN-Schmelze am Sumpf der Quenchkolonne abgezogen wird, verhindert das am Kopf der Quenchkolonne zugefahrene organische Lösungsmittel, dass PDN über Kopf ausgetragen wird. Dieses organische Lösungsmittel wird dabei verdampft und praktisch vollständig über Kopf abgetrennt. Der zuzufahrende Mengenstrom ist entsprechend zu bemessen.

Bezüglich der Zusammensetzung des Quenchkolonnenaustrags ist der Übergang von einer Lösung von PDN in dem organischen Lösungsmittel, erhalten bei tieferer Sumpftemperatur der Quenchkolonne, und einer weitgehend lösungsmittelfreien PDN-Schmelze, erhalten bei höherer Sumpftemperatur der Quenchkolonne, fließend.

Die Temperaturen des Quenchkolonnenaustrags betragen im allgemeinen wie bereits oben beim Quenchschritt beschrieben.

Zum Erhalt einer PDN-Schmelze im Sumpfaustrag der Quenchkolonne kann wie folgt vorgegangen werden:
Die heißen Reaktionsgase aus der Ammonoxidation werden im Sumpf einer Quenchkolonne zugefahren, vgl. Abb. 3. Ein Teil des Sumpfaustrages wird im Kreis geführt und nach Abkühlung auf ca. 165 bis 180°C in etwa in Kolonnenmitte wieder zugefahren. Dabei ist sicherzustellen, dass die Schmelztemperatur nicht unterschritten wird. Der Mengenstrom der Kreislaufschmelze ist so einzustellen, dass die erforderliche thermische Leistung abgeführt werden kann. Die Kolonne ist mit Einbauten, wie z.B. Böden oder Packungen zur Erhöhung der Trennleistung ausgestattet. Am Kopf der Kolonne wird ein organisches Lösungsmittel mit einem Siedepunkt kleiner als dem von PDN zugefahren. Dadurch wird im oberen Teil der Kolonne dampfförmiges PDN kondensiert und gleichzeitig das Lösungsmittel verdampft. Damit wird gewährleistet, dass praktisch kein PDN über Kopf der Quenchkolonne ausgetragen wird. Der Sumpfaustrag besteht aus PDN mit geringen Anteilen des Lösungsmittels sowie Nebenkomponenten aus der Ammonoxidation. Die leichtsiedenden Nebenkomponenten können in einer anschließenden Destillationsstufe abgetrennt werden. Bevorzugt wird die Schmelze jedoch ohne weitere Leichtsiederabtrennung zur Hydrierung gefahren.

### Hydrierung:

Die nach der Leichtsiederabtrennung wie oben beschrieben erhaltene Phthalodinitrilschmelze, bzw. Lösung oder Emulsion wird anschließend der Hydrierung zugeführt.

Eine Abtrennung von Hochsiedern, also von Produkten mit einem Siedepunkt höher als Phthalodinitril (bei gleichem Druck), findet im erfindungsgemäßen Verfahren vor der Hydrierung des PDNs nicht statt.

Für die Hydrierung des Phthalodinitrils zum entsprechenden Xylylendiamin (o-, m- bzw. p-Xylylendiamin) wird dem PDN besonders bevorzugt Ammoniak, bevorzugt in flüssiger Form, zugefügt.

Für die Hydrierung des Phthalodinitrils kann auch ein organisches Lösungsmittel zugesetzt werden. Wird die Hydrierung in Gegenwart von Ammoniak und einem organischen Lösungsmittel durchgeführt, wird bevorzugt zuerst die Lösung oder Suspension im Lösungsmittel hergestellt.
Als Lösungsmittel bevorzugt sind hier NMP, Xylol, Benzylamin, o-, m- oder p-Methyl-benzylamin, Xylylendiamin und Mischungen hiervon.

Eine bevorzugte Ausführungsform besteht in der alleinigen Verwendung von flüssigem Ammoniak als Lösungsmittel.

Das Gewichtsverhältnis im Frischzulauf von eingesetztem Dinitril zu Ammoniak beträgt im allgemeinen 1 : 0,15 bis 1 : 15, vorzugsweise 1 : 0,5 bis 1 : 10, besonders bevorzugt 1 : 1 bis 1 : 5.

Für die Hydrierung können die dem Fachmann für diese Umsetzung bekannten Katalysatoren und Reaktoren (z.B. Festbett- oder Suspensionsfahrweise) sowie Verfahren (kontinuierlich, halbkontinuierlich, diskontinuierlich) angewendet werden.

Bei der Katalysatorfestbettfahrweise ist sowohl die Sumpf- als auch die Rieselfahrweise möglich. Bevorzugt ist eine Rieselfahrweise.

Diesbezüglich wird hiermit z.B. auf die in den Anmeldungen GB-A-852,972 (Äquivalent: DE-A-11 19 285) (BASF AG) und DE-A-12 59 899 (BASF AG) und dem US Patent Nr. 3,069,469 (California Research Corp.) beschriebenen Verfahren verwiesen.

Der Hydrierreaktor kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes. Damit lässt sich eine optimale Verdünnung der Reaktionslösung erreichen, was sich günstig auf die Selektivität auswirkt. Insbesondere kann der Kreislaufstrom mittels eines externen Wärmeüberträgers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist eine einfache und kostengünstige Bauform möglich. Eine Alternative stellt ein gekühlter Rohrbündelreaktor dar.

Bevorzugt sind Katalysatoren, die Kobalt und/oder Nickel und/oder Eisen, als Vollkatalysator oder auf einem inerten Träger, enthalten.

Hierbei liegen die Reaktionstemperaturen im allgemeinen bei 40 bis 150°C, bevorzugt bei 40 bis 120°C.

Der Druck liegt im allgemeinen bei 40 bis 300 bar, bevorzugt 100 bis 200 bar.

### Isolierung des XDAs:

Nach der Hydrierung werden das gegebenenfalls verwendete Lösungsmittel und der gegebenenfalls eingesetzte Ammoniak abdestilliert.

Bevorzugt erfolgt eine Reinigung des Xylylendiamins durch Abdestillation leichtersiedender Nebenprodukte (bei gleichem Druck) über Kopf und destillativer Abtrennung von schwerersiedenden Verunreinigungen über Sumpf.

Besonders bevorzugt ist die Fahrweise, in der man nach der Hydrierung das gegebenenfalls verwendete Lösungsmittel, gegebenenfalls Ammoniak sowie gegebenenfalls leichtersiedende Nebenprodukte über Kopf abdestilliert und danach schwerersiedende Verunreinigungen vom Xylylendiamin destillativ über Sumpf abtrennt.

In einer besonderen Ausführungsform kann die Abtrennung leichter- und schwerersiedender Nebenprodukte auch in einer Seitenabzugs- oder Trennwandkolonne erfolgen, wobei reines Xylylendiamin über einen flüssigen oder gasförmigen Seitenabzug gewonnen wird.

Je nach gewünschter Reinheit wird das Produkt (XDA) zusätzlich mit einem organischen Lösungsmittel, bevorzugt einem aliphatischen Kohlenwasserstoff, insbesondere einem cycloaliphatischen Kohlenwasserstoff, ganz besonders Cyclohexan oder Methylcyclohexan, extrahiert.
Diese Reinigung durch Extraktion kann z.B. gemäß DE-A-1 074 592 erfolgen.

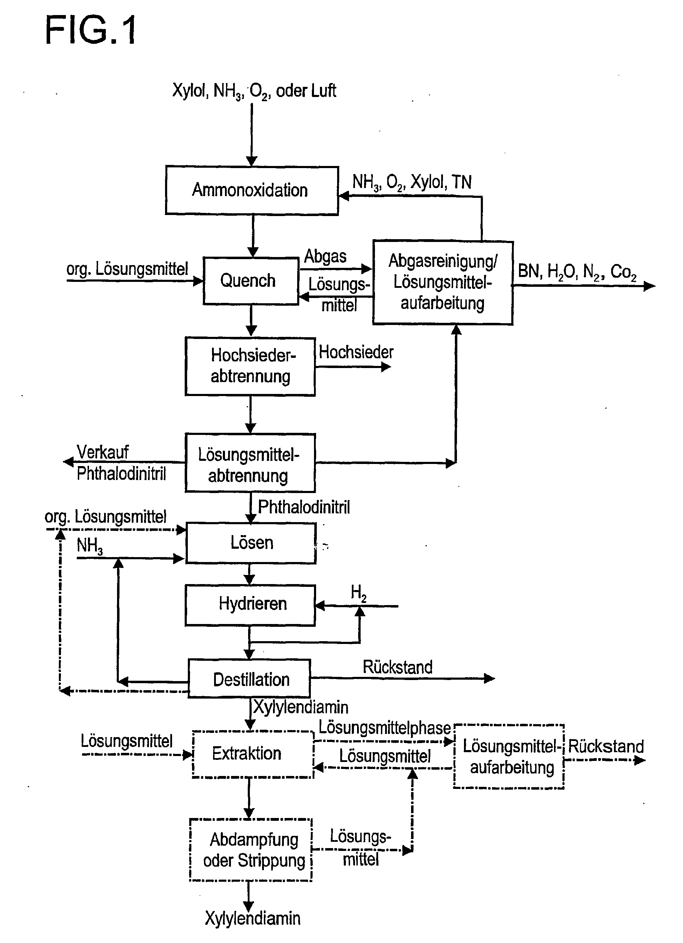

Einen schematischen Überblick über eine bevorzugte Ausgestaltung des erfindungsgemäßen Verfahrens gibt die Abbildung 1 in der Anlage. Die in Abbildung 1 dargestellte Hochsiederabtrennung ist jedoch nicht erfindungsgemäß.
Die optionalen Verfahrensmerkmale ,organisches Lösungsmittel bei der Hydrierung' und ,extraktive XDA-Reinigung' sind gestrichelt gezeichnet.

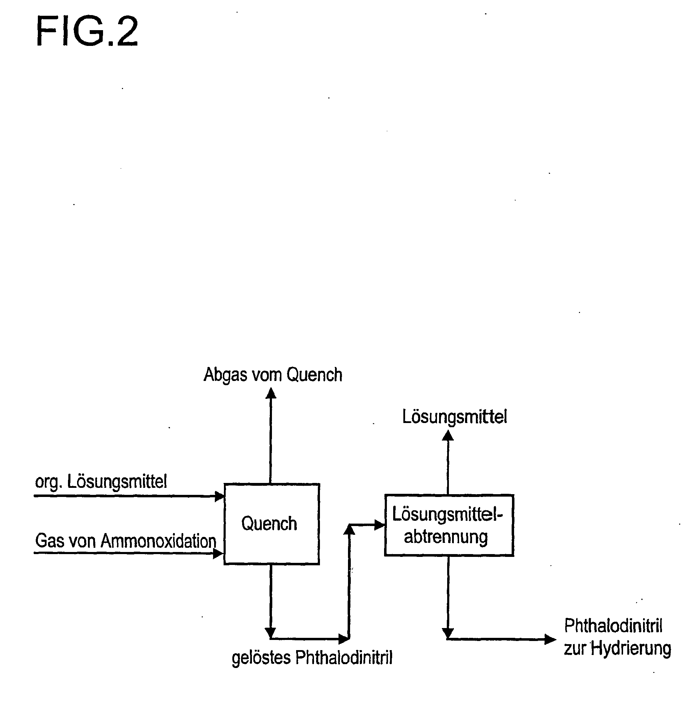

Abbildung 2 zeigt ein Schema des Quenchschritts mit darauffolgender Leichtsiederabtrennung (inkl. Quenchlösungsmittel).

Abbildung 3 zeigt ein Schema der Kombination des Quenchschrittes mit der Leichtsiederabtrennung (inkl. Quenchlösungsmittel) in einer Kolonne.

### Beispiele

### Beispiel 1: (Vergleichsbeispiel)

Ammonoxidation von m-Xylol, anschließendes Quenchen der Reaktionsgase mit Tolunitril als Lösungsmittel, Leichtsiederabtrennung und Hydrierung des in der Ammonoxidationsstufe entstandenen IPDNs (vergl. Verfahrensschema in Abbildung 1)

Ein Katalysator der Zusammensetzung V₄Sb₃W_{0,4}Cs_{0,2} auf Steatit wurde als Festbett in einen Rohrreaktor eingebaut. Die Apparatur wurde von außen auf 400°C aufgeheizt. Dem Reaktor wird verdampftes m-Xylol, gasförmiger Ammoniak, Luft und Stickstoff zugefahren (NH₃ / m-Xylol = 8 mol / 1 mol; O₂ / m-Xylol = 4 mol / 1 mol). Der vordere Teil des Reaktors war mit einer Inertschüttung gefüllt, so dass die Einsatzstoffe vorgemischt und auf 400°C vorgeheizt die Reaktionszone erreichten. Im Reaktor herrschte ein leichter Überdruck von 20 bis 30 mbar. Die Hot-Spot Temperatur erreichte 450°C. Man erhielt bei einem Umsatz (U) von m-Xylol von 79 % eine Selektivität (S) zu IPDN von 68 %.

Das aus dem Reaktor austretende Gasgemisch wird in einer Kolonne mit Tolunitril gequencht. Aus der Quenchkolonne wird bei 120°C eine Lösung von IPDN in Tolunitril ausgetragen, welche 1 Gew.-% m-Xylol, 0,3 Gew.-% Wasser, 0,1 Gew.-% Benzonitril, 80 Gew.-% Tolunitril und 18,7 Gew.-% IPDN enthält. Über Kopf der Quenchkolonne werden nicht umgesetzte Reaktionsgase und Inertgase sowie nicht umgesetztes m-Xylol sowie etwas Tolunitril gasförmig abgezogen. Dieses Gas kann aufgearbeitet werden, um die Wertstoffe (insbesondere NH₃, m-Xylol, sowie Tolunitril) in die Reaktionsstufe bzw. in den Quenchkreis zurückzuführen. Inerte und Begleitkomponenten (H₂O, Benzonitril, N₂, CO₂, etc.) werden aus der Aufarbeitungsstufe ausgeschleust.

Die nach dem Quench erhaltene Lösung von IPDN in Tolunitril wird bei 100 mbar (abs.) einer der mittleren Stufen einer Destillationskolonne zugefahren. Über Kopf werden bei 57°C Xylol, Tolunitril, Benzonitril und Wasser abgetrennt. Über Sumpf wird IPDN mit 0,1 Gew.-% Tolunitril bei 195°C abgezogen. Der Kopfabzugsstrom kann aufgearbeitet und zur Ammonoxidation bzw. zum Quenchkreis zurückgeführt werden.

27 Gew.-% IPDN wurde mit 73 Gew.-% NMP gemischt und in einem kontinuierlich betriebenen 70 ml-Rohrreaktor an einem Kobalt-Vollkontakt bei 80°C und 190 bar hydriert. Über den Katalysator wurden stündlich 70 g IPDN-Lösung sowie 90 g Ammoniak geleitet. Die Ausbeute an MXDA betrug 96 % bezogen auf eingesetztes IPDN.

In einer anschließenden Batchdestillation wurden zuerst noch gelöster Ammoniak und danach NMP und leichtsiedende Nebenkomponenten abgetrennt. Nach Abtrennung der hochsiedenden Verunreinigungen wurde MXDA in einer Reinheit von mehr als 99,9 Gew.-% erhalten.

### Beispiel 2 (alternative Hydriereinstellung):

Eine Mischung bestehend aus 27 Gew.-% IPDN und 73 Gew.-% NMP, die aus den reinen Komponenten zusammengemischt wurde, wurde in einem kontinuierlich betriebenen 70 ml-Rohrreaktor an einem Kobalt-Vollkontakt bei 80°C und 190 bar hydriert. Über den Katalysator wurden stündlich 70 g IPDN-Lösung sowie 54 g Ammoniak geleitet. Die gleiche Volumenmenge wird als Lösemittel im Kreis gefahren. Die Ausbeute an MXDA betrug 95,5 % bezogen auf eingesetztes IPDN.

### Beispiel 3 (alternative Hydriereinstellung):

Eine Mischung bestehend aus 15 Gew.-% IPDN und 85 Gew.-% MXDA, die aus den reinen Komponenten zusammengemischt wurde, wurde in einem kontinuierlich betriebenen 70 ml-Rohrreaktor an einem Kobalt-Vollkontakt bei 60°C und 190 bar hydriert. Über den Katalysator wurden stündlich 117 g IPDN-Lösung sowie 150 g Ammoniak geleitet. Ein Viertel der Volumenmenge wird als Lösemittel im Kreis gefahren. Die Ausbeute an MXDA betrug 92 % bezogen auf eingesetztes IPDN.

In anschließenden Destillationsschritten wurden zuerst Ammoniak und danach leichtsiedende Nebenkomponenten abgetrennt. Nach Abtrennung der hochsiedenden Verunreinigungen über Sumpf wurde MXDA als Kopfprodukt einer Destillationskolonne in einer Reinheit von mehr als 99,9 Gew.-% erhalten.

### Beispiel 4 (alternative Hydriereinstellung):

30 g IPDN sowie 5 g Raney-Nickel wurden im Rührautoklaven vorgelegt. Nach Zugabe von 66 g Ammoniak wurden 50 bar Wasserstoff aufgepresst und auf 100°C aufgeheizt. Durch Wasserstoffnachpressen wurde ein Gesamtdruck von 100 bar für 5 Stunden gehalten. Die Umsetzung von IPDN war quantitativ, wobei eine Ausbeute von 94 % bezogen auf eingesetztes IPDN erhalten wurde.

(Die oben angegebenen Daten des Quenchschrittes und der Reindestillation von IPDN sind die Ergebnisse einer thermodynamischen Simulation. Dabei wurde der Quench als Apparat gerechnet, in dem thermodynamisches Gleichgewicht zwischen Gas- und Flüssigphase herrscht. Neben den Reinstoffdaten der beteiligten Komponenten wurden bei der Berechnung reale Binärdaten verwendet. Derartige Berechnungen können mit kommerziellen Rechenprogrammen, hier: Aspen Plus, die dem Fachmann geläufig sind, durchgeführt werden).

### Beispiel 5:

### Untersuchungen zur Löslichkeit von IPDN in verschiedenen Lösungsmitteln

Die Löslichkeit von IPDN in NMP beträgt bei 60°C ca. 26 Gew.-% und bei 90°C ca. 41 Gew.-%.
Pseudocumol erreicht bei 90°C lediglich eine Löslichkeit von 20 Gew.-% und Mesitylen lediglich von 12 Gew.-%.
Bei 60°C liegt die Löslichkeit von IPDN in Mesitylen oder Pseudocumol jeweils unter 10 Gew.-%.

### Beispiel 6:

Ein Katalysator der Zusammensetzung V₄Sb₃K_{0,4}Ba_{0,2} auf Steatit wurde als Festbett in einen Rohrreaktor eingebaut. Die Apparatur wurde von außen auf 415°C aufgeheizt. Dem Reaktor wurde verdampftes m-Xylol, gasförmiger Ammoniak und Luft zugefahren (NH₃ / m-Xylol = 14 mol / 1 mol; O₂ / m-Xylol = 4 mol / 1 mol). Der Katalysator der ersten Hälfte des Reaktors war mit 70 Gew.% Steatitkugeln verdünnt, die zweite Hälfte mit 40 Gew.%. Im Reaktor herrschte ein leichter Überdruck von 0,02 bar. Die Hot-Spot-Temperatur erreichte 430°C. Man erhielt bei einem Umsatz von m-Xylol von 88 % eine Selektivität zu IPDN von 71 %.

Das heiße Quenchgas wird dem Sumpf einer Quenchkolonne zugefahren. Im unteren Teil der Kolonne wird eine Schmelze von IPDN über einen Wärmeübertrager im Kreis gefahren und bei einer Temperatur von 165°C der Kolonne auf der 4. theoretischen Stufe wieder zugeführt. Am Kopf der Kolonne wird frisches m-Tolunitril bei 20°C zugeführt. Das IPDN wird von der umlaufenden Schmelze absorbiert und über Sumpf bei 198°C mit einer Reinheit von mehr als 99 Gew.-% ausgetragen. An Nebenkomponenten sind m-Xylol (640 Gew.-ppm), Wasser (0,15 Gew.-%) sowie 0,55 Gew.-% m-Tolunitril vorhanden. Im oberen Teil wird das vom unteren Teil der Kolonne aufsteigende Gasgemisch mit Tolunitril im Gegenstrom gewaschen, wobei das IPDN vollständig kondensiert wird. Über Kopf werden bei 140°C die Reaktionsgase aus der Ammonoxidation zusammen mit dem Lösungsmittel m-Tolunitril gasförmig ausgetragen. Dieser Gasstrom enthält praktisch kein IPDN mehr. Die über Sumpf ausgetragene Schmelze kann direkt zur Hydrierung eingesetzt werden.

(Die angegebenen Daten des Quenchschrittes sind die Ergebnisse einer thermodynamischen Simulation, wie oben beschrieben).

## Patentansprüche

1. Verfahren zur Herstellung von ortho-, meta- oder para-Xylylendiamin umfassend die Schritte
Ammonoxidation von ortho-, meta- oder para-Xylol zu o-Phthalodinitril, Iso- oder Terephthalodinitril und Hydrierung des Phthalodinitrils,
**dadurch gekennzeichnet, dass** das dampfförmige Produkt der Ammonoxidationsstufe direkt mit einem flüssigen organischen Lösungsmittel, welches einen niedrigeren Siedepunkt als das Phthalodinitril hat, oder mit geschmolzenem Phthalodinitril in Kontakt gebracht wird (Quench),
aus der erhaltenen Quenchlösung oder -suspension bzw. Phthalodinitrilschmelze Komponenten mit einem Siedepunkt niedriger als Phthalodinitril (Leichtsieder) abgetrennt werden,
vor der Hydrierung des Phthalodinitrils keine Produkte mit einem Siedepunkt höher als Phthalodinitril (Hochsieder) abgetrennt werden und
die Hydrierung in Abwesenheit eines organischen Lösungsmittels durchgeführt wird.

2. Verfahren nach Anspruch 1 zur Herstellung von meta-Xylylendiamin umfassend die Schritte Ammonoxidation von meta-Xylol zu Isophthalodinitril und Hydrierung des Isophthalodinitrils.

3. Verfahren nach den Ansprüchen 1 oder 2, **dadurch gekennzeichnet, dass** als flüssiges organisches Lösungsmittel für den Quench ein aromatischer Kohlenwasserstoff, eine heterocyclische Verbindung, ein aromatisches Nitril und/oder ein heterocyclisches Nitril eingesetzt wird.

4. Verfahren nach den Ansprüchen 1 oder 2, **dadurch gekennzeichnet, dass** als flüssiges organisches Lösungsmittel für den Quench Tolunitril, Benzonitril und/oder N-Methyl-2-pyrrolidon (NMP) eingesetzt wird.

5. Verfahren nach einem der vorhergehenden Ansprüche, **dadurch gekennzeichnet, dass** bei dem Quench mit einem flüssigen organischen Lösungsmittel die Temperatur des Quenchaustrags 40 bis 180°C und bei dem Quench mit geschmolzenem Phthalodinitril die Temperatur des Quenchaustrags 165 bis 220°C beträgt.

6. Verfahren nach einem der vorhergehenden Ansprüche, **dadurch gekennzeichnet, dass** die teilweise oder vollständige Abtrennung der Leichtsieder aus der erhaltenen Quenchlösung oder -suspension bzw. Phthalodinitrilschmelze destillativ über Kopf erfolgt, während Phthalodinitril zusammen mit Produkten mit einem Siedepunkt höher als Phthalodinitril (Hochsieder) über Sumpf abgetrennt werden.

7. Verfahren nach einem der Ansprüche 1 bis 5, **dadurch gekennzeichnet, dass** der Quench des dampfförmigen Produkts der Ammonoxidationsstufe so in einer Kolonne durchgeführt wird, dass Reaktionsgase und Leichtsieder teilweise oder vollständig über Kopf und Phthalodinitril zusammen mit Produkten mit einem Siedepunkt höher als Phthalodinitril (Hochsieder) über Sumpf abgetrennt werden.

8. Verfahren nach einem der vorhergehenden Ansprüche, **dadurch gekennzeichnet, dass** die Ammonoxidation bei Temperaturen von 300 bis 500°C an einem Katalysator enthaltend V, Sb und/oder Cr, als Vollkatalysator oder auf einem inerten Träger, durchgeführt wird.

9. Verfahren nach einem der vorhergehenden Ansprüche, **dadurch gekennzeichnet, dass** die Hydrierung in Gegenwart von Ammoniak durchgeführt wird.

10. Verfahren nach einem der vorhergehenden Ansprüche, **dadurch gekennzeichnet, dass** die Hydrierung bei Temperaturen von 40 bis 150°C an einem Katalysator enthaltend Ni, Co und/oder Fe, als Vollkatalysator oder auf einem inerten Träger, durchgeführt wird.

11. Verfahren nach einem der vorhergehenden Ansprüche, **dadurch gekennzeichnet, dass** nach der Hydrierung eine Reinigung des Xylylendiamins durch Abdestillation des gegebenenfalls eingesetzten Lösungsmittels und Ammoniaks sowie gegebenenfalls leichtersiedender Nebenprodukte über Kopf und destillativer Abtrennung von schwerersiedenden Verunreinigungen über Sumpf erfolgt.

12. Verfahren nach einem der vorhergehenden Ansprüche, **dadurch gekennzeichnet, dass** man nach der Hydrierung das gegebenenfalls eingesetzte Lösungsmittel und Ammoniak sowie gegebenenfalls leichtersiedende Nebenprodukte über Kopf abdestilliert und danach schwerersiedende Verunreinigungen vom Xylylendiamin destillativ über Sumpf abtrennt.

13. Verfahren nach einem der beiden vorhergehenden Ansprüche, **dadurch gekennzeichnet, dass** das Xylylendiamin nach der Destillation zur weiteren Reinigung mit einem organischem Lösungsmittel extrahiert wird.

14. Verfahren nach dem vorhergehenden Anspruch, **dadurch gekennzeichnet, dass** man zur Extraktion Cyclohexan oder Methylcyclohexan verwendet.

## Claims

1. A process for preparing ortho-, meta- or para-xylylenediamine, comprising the steps of ammoxidizing ortho-, meta- or para-xylene to o-phthalonitrile, iso- or terephthalonitrile and hydrogenating the phthalonitrile, which comprises contacting the vaporous product of the ammoxidation stage directly with a liquid organic solvent which has a lower boiling point than the phthalonitrile or with molten phthalonitrile (quench),
removing components having a boiling point lower than phthalonitrile (low boilers) from the resulting quench solution or suspension or phthalonitrile melt
before the hydrogenation of the phthalonitrile, not removing any products having a boiling point higher than phthalonitrile (high boilers).

2. The process according to claim 1 for preparing meta-xylylenediamine, comprising the steps of ammoxidizing meta-xylene to isophthalonitrile and hydrogenating the isophthalonitrile.

3. The process according to either of claims 1 and 2, wherein the liquid organic solvent used for the quench is an aromatic hydrocarbon, a heterocyclic compound, an aromatic nitrile and/or a heterocyclic nitrile.

4. The process according to either of claims 1 and 2, wherein the liquid organic solvent used for the quench is tolunitrile, benzonitrile and/or N-methyl-2-pyrrolidone (NMP).

5. The process according to any of the preceding claims, wherein, in the quench with a liquid organic solvent, the temperature of the quench effluent is from 40 to 180°C, and, in the quench with molten phthalonitrile, the temperature of the quench effluent is from 165 to 220°C.

6. The process according to any of the preceding claims, wherein the low boilers are partly or fully removed from the resulting quench solution or suspension or phthalonitrile melt by distillation via the top, while phthalonitrile is removed via the bottom together with products having a boiling point higher than phthalonitrile (high boilers).

7. The process according to any of claims 1 to 5, wherein the quench of the vaporous product of the ammoxidation stage is carried out in a column in such a way that reaction gases and low boilers are partly or fully removed via the top and phthalonitrile together with products having a boiling point higher than phthalonitrile (high boilers) are removed via the bottom.

8. The process according to any of the preceding claims, wherein the ammoxidation is carried out at temperatures of from 300 to 500°C over a catalyst containing V, Sb and/or Cr, as an unsupported catalyst or on an inert support.

9. The process according to any of the preceding claims, wherein the hydrogenation is carried out in the presence of ammonia.

10. The process according to any of the preceding claims, wherein the hydrogenation is carried out at temperatures of from 40 to 150°C over a catalyst containing Ni, Co and/or Fe, as an unsupported catalyst or on an inert support.

11. The process according to any of the preceding claims, wherein, after the hydrogenation, the xylylenediamine is purified by distilling off any solvent used and ammonia, and also any relatively low-boiling by-products, via the top and distillatively removing relatively high-boiling impurities via the bottom.

12. The process according to any of the preceding claims, wherein, after the hydrogenation, any solvent used and ammonia, and also any relatively low-boiling by-products, are distilled off via the top and, afterwards, any relatively high-boiling impurities are removed from the xylylenediamine by distillation via the bottom.

13. The process according to either of the two preceding claims, wherein the xylylenediamine, after the distillation, is extracted for further purification with an organic solvent.

14. The process according to the preceding claim, wherein cyclohexane or methylcyclohexane are used for the extraction.

## Revendications

1. Procédé de préparation d'ortho-xylylènediamine, méta-xylylènediamine ou para-xylylènediamine comprenant les étapes
d'ammonoxydation d'ortho-xylène, de méta-xylène ou de para-xylène en o-phtalodinitrile, isophtalodinitrile ou téréphtalodinitrile et d'hydrogénation du phtalodinitrile,
**caractérisé en ce que** le produit en forme de vapeur de l'étape d'ammonoxydation est amené directement en contact (refroidissement brusque) avec un solvant organique liquide, qui a un point d'ébullition inférieur au phtalodinitrile, ou avec du phtalodinitrile fondu,
des composants ayant un point d'ébullition inférieur au phtalodinitrile (substances à bas point d'ébullition) étant séparés de la solution ou suspension de refroidissement brusque obtenue ou respectivement de la masse fondue de phtalodinitrile,
aucun produit ayant un point d'ébullition supérieur au phtalodinitrile (substances à haut point d'ébullition) n'étant séparé avant l'hydrogénation du phtalodinitrile, et
l'hydrogénation étant effectuée en l'absence d'un solvant organique.

2. Procédé suivant la revendication 1 pour la préparation de méta-xylylènediamine, comprenant les étapes d'ammonoxydation de méta-xylène en isophtalodinitrile et d'hydrogénation de l'isophtalodinitrile.

3. Procédé suivant les revendications 1 ou 2, **caractérisé en ce que**, comme solvant organique liquide pour le refroidissement brusque, on met en oeuvre un hydrocarbure aromatique, un composé hétérocyclique, un nitrile aromatique et/ou un nitrile hétérocyclique.

4. Procédé suivant les revendications 1 ou 2, **caractérisé en ce que**, comme solvant organique liquide pour le refroidissement brusque, on met en oeuvre du toluonitrile, du benzonitrile et/ou de la N-méthyl-2-pyrrolidone (NMP).

5. Procédé suivant l'une des revendications précédentes, **caractérisé en ce que**, lors du refroidissement brusque avec un solvant organique liquide, la température de l'effluent du refroidissement brusque est de 40 à 180°C et, lors du refroidissement brusque avec du phtalodinitrile fondu, la température de l'effluent du refroidissement brusque est de 165 à 220°C.

6. Procédé suivant l'une des revendications précédentes, **caractérisé en ce que** la séparation partiel ou total des substances à bas point d'ébullition hors de la solution ou suspension de refroidissement brusque obtenue ou de la masse fondue de phtalodinitrile a lieu par distillation par la tête, tandis que du phtalodinitrile est séparé conjointement aux produits ayant un point d'ébullition supérieur au phtalodinitrile (substances à haut point d'ébullition) par le fond.

7. Procédé suivant l'une des revendications 1 à 5, **caractérisé en ce que** le refroidissement brusque du produit en forme de vapeur de l'étape d'ammonoxydation est effectué dans une colonne, de façon que des gaz réactionnels et des substances à bas point d'ébullition soient séparés partiellement ou totalement par la tête et du phtalodinitrile soit séparé conjointement aux produits ayant un point d'ébullition supérieur au phtalodinitrile (substances à haut point d'ébullition) par le fond.

8. Procédé suivant l'une des revendications précédentes, **caractérisé en ce que** l'ammonoxydation est effectuée à des températures de 300 à 500°C sur un catalyseur contenant V, Sb et/ou Cr, sous la forme de catalyseur massique ou sur un support inerte.

9. Procédé suivant l'une des revendications précédentes, **caractérisé en ce que** l'hydrogénation est effectuée en présence d'ammoniac.

10. Procédé suivant l'une des revendications précédentes, **caractérisé en ce que** l'hydrogénation est effectuée à des températures de 40 à 150°C sur un catalyseur contenant Ni, Co et/ou Fe, sous la forme de catalyseur massique ou sur un support inerte.

11. Procédé suivant l'une des revendications précédentes, **caractérisé en ce que**, après l'hydrogénation, une purification de la xylylènediamine a lieu par distillation, par la tête, de l'ammoniac et du solvant éventuellement mis en oeuvre ainsi qu'éventuellement des produits secondaires à point d'ébullition inférieur, et par séparation par distillation, par le fond, des impuretés à point d'ébullition supérieur.

12. Procédé suivant l'une des revendications précédentes, **caractérisé en ce que**, après l'hydrogénation, on sépare, de la xylylènediamine, par distillation, par la tête, l'ammoniac et le solvant éventuellement mis en oeuvre ainsi qu'éventuellement des produits secondaires à point d'ébullition inférieur et ensuite on sépare par distillation, par le fond, des impuretés à point d'ébullition supérieur.

13. Procédé suivant l'une des deux revendications précédentes, **caractérisé en ce que** la xylylènediamine est, après la distillation, extraite par un solvant organique pour une purification ultérieure.

14. Procédé suivant la revendication précédente, **caractérisé en ce que**, pour l'extraction, on utilise du cyclohexane ou du méthylcyclohexane.
